(19) Europäisches Patentamt
European Patent Office
Office européen des brevets

(11) EP 3 633 049 A1

(12) EUROPEAN PATENT APPLICATION
published in accordance with Art. 153(4) EPC

(43) Date of publication:
**08.04.2020 Bulletin 2020/15**

(21) Application number: **18809563.2**

(22) Date of filing: **17.05.2018**

(51) Int Cl.:
***C12Q 1/6876*** *(2018.01)* ***C12N 15/09*** *(2006.01)*

(86) International application number:
**PCT/JP2018/019095**

(87) International publication number:
**WO 2018/221240 (06.12.2018 Gazette 2018/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.05.2017 JP 2017105202**

(71) Applicant: **NIPPON STEEL ECO-TECH CORPORATION**
**Tokyo 104-0031 (JP)**

(72) Inventors:
- **KURATA, Shinya**
**Tokyo 1040031 (JP)**
- **TAKADA, Katsumi**
**Tokyo 1040031 (JP)**

(74) Representative: **J A Kemp LLP**
**14 South Square**
**Gray's Inn**
**London WC1R 5JJ (GB)**

# (54) NOVEL FLUORESCENCE QUENCHING PROBE FOR MEASURING NUCLEIC ACID

(57) The present invention provides a QProbe that can be fluorescently labeled at a lower cost. A nucleic acid probe labeled with a fluorescent dye whose emission is reduced when the probe is hybridized with a target nucleic acid is provided. The probe is labeled with a fluorescent dye at its 5' terminal site via an ssH amino linker as shown below or at its 3' terminal site via a CA amino linker as shown below. The base sequence of the nucleic acid probe is so designed that, when the probe is hybridized with a target nucleic acid, at least one G (guanine) exists in the base sequence of the target nucleic acid at a position 1 to 3 bases apart from the base at the terminal site where the probe and the target nucleic acid are hybridized with each other.

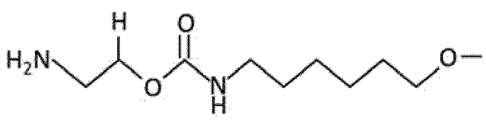

ssH amino linker

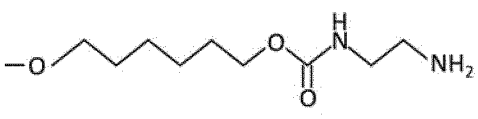

CA amino linker

EP 3 633 049 A1

Fig. 3

| | 5' end | 3' end |
|---|---|---|
| Existing amino linker | 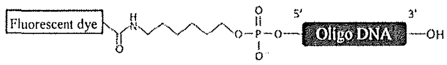 | 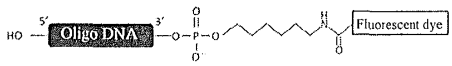 |
| Novel amino linker | 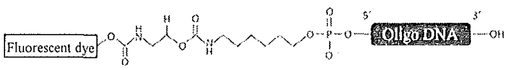 | 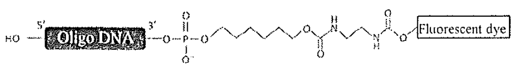 |

## Description

TECHNICAL FIELD

The present invention relates to a genetic analysis technique which comprises using a nucleic acid probe labeled with a fluorescent dye to analyze a target gene qualitatively and quantitatively based on a fluorescence character change resulting from the hybridization of the above nucleic acid probe and the target gene in a homogeneous solution system.

BACKGROUND ART

Genetic analysis methods using a nucleic acid probe whose fluorescence characters (such as fluorescence intensity and fluorescence spectrum) change upon hybridization with a target nucleic acid in a homogeneous solution system (hereinafter, referred to as "homogeneous solution system probe") have become popular. Commonly used as the homogeneous solution system probe are TaqMan probe (Non-Patent Document No. 1), Molecular beacons (Non-Patent Document No. 2), QProbe (Non-Patent Document No. 3) and the like. Among these, QProbe is a homogeneous solution system probe based on a fluorescence quenching phenomenon resulting from electron transfer between fluorescent dye and guanine base (Non-Patent Document No. 4), and the fluorescence of this probe is quenched upon hybridization with a target nucleic acid. Detection of the target nucleic acid is possible by monitoring this fluorescence quenching. Further, the fluorescence of QProbe is quenched via interaction with the guanine in the target nucleic acid, so the probe need be labeled with only one fluorescent dye and features a very simple structure compared to other probes. Thus, QProbe has an advantage of being low in production cost.

PRIOR ART LITERATURE

Non-Patent Documents

Non-Patent Document No. 1: Detection of specific polymerase chain reaction product by utilizing the 5'----3' exonuclease activity of Thermus aquaticus DNA polymerase., Holland PM, Abramson RD, Watson R, Gelfand DH., Proc Natl Acad Sci USA., 1991 Aug 15;88(16):7276-80.

Non-Patent Document No. 2: Molecular beacons: probes that fluoresce upon hybridization, Tyagi S, Kramer FR., Nat Biotechnol., 1996 Mar;14(3):303-8.b

Non-Patent Document No. 3: Fluorescent quenching-based quantitative detection of specific DNA/RNA using a BODIPY((R)) FL-labeled probe or primer., Kurata S, Kanagawa T, Yamada K, Torimura M, Yokomaku T, Kamagata Y, Kurane R., Nucleic Acids Res., 2001 Mar 15;29(6):E34.

Non-Patent Document No. 4: Fluorescence-quenching phenomenon by photoinduced electron transfer between a fluorescent dye and a nucleotide base., Torimura M, Kurata S, Yamada K, Yokomaku T, Kamagata Y, Kanagawa T, Kurane R., Anal Sci., 2001 Jan;17(1):155-60.

SUMMARY OF THE INVENTION

PROBLEM FOR SOLUTION BY THE INVENTION

Since the above-described quenching phenomenon is observed most markedly when a probe's terminal base cytosine is labeled with a fluorescent dye having such a property that its fluorescence is quenched via interaction with guanine (Non-Patent Document No. 3), QProbe in most cases has such a structure that the terminal cytosine base is fluorescently labeled. Fluorescent labeling of a terminal base of QProbe is performed by first synthesizing an oligo DNA in which the end of the terminal cytosine (i.e., the phosphate group at the 5' end if the terminal cytosine base is located on the 5' terminal side; or the phosphate group at the 3' end if the terminal cytosine base is located on the 3' terminal side) is labeled with an amino group-containing linker (hereinafter, referred to as "amino linker") and then labeling the amino group in the amino linker with a fluorescent dye. As fluorescent dyes for use in such fluorescence labeling via amino group, those having functional groups of high reactivity with amino group (such as succinimidyl ester group, sulfosuccinimidyl ester group, TFP (tetrafluorophenyl) ester group, and STP (sulfotetrafluorophenyl) ester group) may generally be utilized. As the amino linker, a structure having an amino group at one end of its carbon chain may generally be utilized (see Fig. 1).

As described above, QProbe needs only one fluorescent dye for its modification, so its production cost is

generally lower than that of other homogeneous solution system probes. On the other hand, the reactivity between the amino group and the fluorescent dye is generally low, so the fluorescent dye must be added in great excess with respect to the amino group. Since the cost of fluorescent dyes is generally high, the production cost of QProbe can be further lowered if it is possible to improve the efficiency of reactivity between amino group and fluorescent dye.

It is therefore an object of the present invention to provide a QProbe which requires an even lower cost in fluorescence labeling.

MEANS TO SOLVE THE PROBLEM

Recently, novel amino linkers (5' ssH amino linker and 3' amino CA linker) designed to have higher reactivity with fluorescent dyes were reported (Japanese Patent No. 4336820). The structures of oligo DNA termini at which these novel amino linkers have been introduced are respectively shown in Fig. 2. When the novel amino linkers are used, the efficiency of reaction between the amino group of each linker and a fluorescent dye is remarkably improved, making it possible to achieve considerable reduction in the amount of expensive fluorescent dyes to be used. Accordingly, if the novel amino linkers can be used in the fluorescence labeling of QProbe, the cost for fluorescent reagent (which accounts for a substantial portion of the reagents cost) can be greatly reduced, compared to the case where existing amino linkers are used.

However, when the novel amino linkers are used, the molecular structures after labeling with a fluorescent dye are different from the corresponding molecular structures for the case where the existing amino linkers are used (Fig. 3); hence, there was a concern that use of the novel amino linkers might cause a performance drop of QProbe in terms of its function.

Under these circumstances, the present inventors synthesized QProbes to which the novel amino linkers were actually applied (hereinafter, referred to as "novel QProbes") and compared these novel QProbes with conventional QProbes to which the existing amino linkers were applied (hereinafter, referred to as "existing QProbes") in terms of their functions (i.e., efficiency of fluorescence quenching upon hybridization with a target nucleic acid). As a result, the present inventors confirmed that the novel QProbes had functionality equivalent or superior to that of the existing QProbes. The present invention has been achieved based on these findings.

The present invention provides a nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 5' terminal site via an ssH amino linker as shown below, and (iii) the nucleotide sequence of the probe is so designed that, when the probe is hybridized with the target nucleic acid, at least one G (guanine) exists in the base sequence of the target nucleic acid at a position 1 to 3 bases apart from the base at the terminal site where the probe and the target nucleic acid are hybridized with each other.

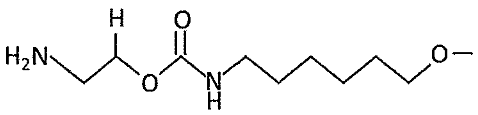 ssH amino linker

The present invention also provides a nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 3' terminal site via a CA amino linker as shown below, and (iii) the base sequence of the probe is so designed that, when the probe is hybridized with the target nucleic acid, at least one G (guanine) exists in the base sequence of the target nucleic acid at a position 1 to 3 bases apart from the base at the terminal site where the probe and the target nucleic acid are hybridized with each other.

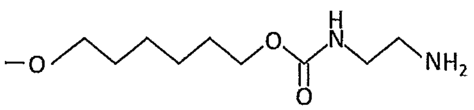 CA amino linker

Further, the present invention provides a nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 5' terminal site via an ssH amino linker as shown below, and (iii) the base sequence of the probe is so designed that, when the probe is hybridized with the target nucleic acid, base pairs in a probe-nucleic acid hybrid complex form at least one G (guanine) and C (cytosine) pair at the terminal site.

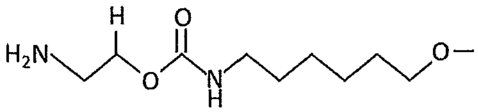

ssH amino linker

Still further, the present invention provides a nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 3' terminal site via a CA amino linker as shown below, and (iii) the base sequence of the probe is so designed that, when the probe is hybridized with the target nucleic acid, base pairs in a probe-nucleic acid hybrid complex form at least one G (guanine) and C (cytosine) pair at the terminal site.

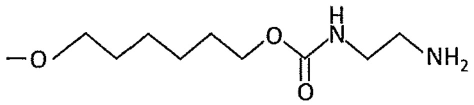

CA amino linker

The present invention also provides the above-described nucleic acid probe for measuring a nucleic acid, wherein the terminal base at which the probe is labeled with the fluorescent dye is C (cytosine).

The present invention also provides the above-described nucleic acid probe for measuring a nucleic acid, wherein the probe is labeled with at least one fluorescent dye selected from the group consisting of Pacific Blue, ATTO465, BODIPY™ FL, 5-CR 6G, 6-TAMRA, ATTO655, ATTO680 and ATTO700.

Further, the present invention provides a method of measuring a nucleic acid using a nucleic acid probe labeled with a fluorescent dye, wherein (i) the probe is labeled with a fluorescent dye whose emission is reduced when the probe is hybridized with a target nucleic acid and (ii) a decrease in the emission from the fluorescent dye is measured, the method being characterized by using the above-described nucleic acid probe for measuring a nucleic acid.

## EFFECT OF THE INVENTION

According to the present invention, nucleic acid probes that retain the performance of conventional QProbe can be produced at a lower cost.

The present specification encompasses the contents disclosed in the specification and /or the drawings of Japanese Patent Application No. 2017-105202 based on which the present patent application claims priority.

## BRIEF DESCRIPTION OF THE DRAWINGS

Fig. 1 shows structural formulas for the case where an existing amino linker (C6 amino linker) has been introduced at one end of an oligo DNA.

Fig. 2 shows structural formulas for the case where a novel amino linker has been introduced at one end of an oligo DNA.

Fig. 3 shows how the vicinity of one end of oligo DNA after fluorescence labeling varies in structural formula depending on the difference in the type of amino linker.

Fig. 4 shows the structural formula for QProbes synthesized in Example 1.

Fig. 5 shows the maximum fluorescence quenching rates (%) of the QProbes synthesized in Example 1.

Fig. 6-1 shows the fluorescence quenching rates of QProbes using a novel amino linker as compared with those of QProbes using a conventional amino linker (in the case where 5' terminal cytosine was fluorescently labeled).

Fig. 6-2 shows the fluorescence quenching rates of QProbes using a novel amino linker as compared with those of QProbes using a conventional amino linker (in the case where 3' terminal cytosine was fluorescently labeled).

Fig. 7-1 shows the proportion of aminated oligo DNA that reacted with a fluorescent dye for fluorescent labeling at 5' end in QProbes using a novel amino linker and QProbes using a conventional amino linker.

Fig. 7-2 shows the proportion of aminated oligo DNA that reacted with a fluorescent dye for fluorescent labeling at 3' end in QProbes using a novel amino linker and QProbes using a conventional amino linker.

## BEST MODES FOR CARRYING OUT THE INVENTION

Hereinbelow, the present invention will be described more specifically with reference to preferred embodiments. As used herein, terms such as DNAs, RNAs, cDNAs, mRNAs, rRNAs, XTPs, dXTPs, NTPs, dNTPs, nucleic acid probes,

helper nucleic acid probes (or nucleic acid helper probes, or simply helper probes), to hybridize, hybridization, intercalators, primers, annealing, extending reactions, thermal denaturing reactions, nucleic acid melting curves, PCR, RT-PCR, RNA-primed PCR, stretch PCR, reverse PCR, PCR using Alu sequence(s), multiple PCR, PCR using mixed primers, PCR using PNA, hybridization assays, FISH (fluorescent *in situ* hybridization) assays, PCR methods (polymerase chain assays), LCR methods (ligase chain reactions), SD methods (strand displacement assays), competitive hybridization, DNA chips, nucleic acid detecting (gene detecting) devices, SNP (single nucleotide polymorphism), co-cultivation systems of plural microorganisms, etc. have the same meanings as the corresponding terms currently in general use in such fields as molecular biology, genetic engineering, and bioengineering. In a method of measuring a target nucleic acid using a nucleic acid probe labeled with a fluorescent dye according to the present invention, the emission from the fluorescent dye decreases when the nucleic acid probe is hybridized with the target nucleic acid and the resulting change from the value before hybridization is measured.

As used herein, the expression "measurement of a target nucleic acid" means quantification or quantitative detection of the target nucleic acid or mere detection the target nucleic acid. The expression "method of measuring a nucleic acid using a nucleic acid probe labeled with a fluorescent dye" refers to hybridization assays, FISH methods (fluorescent *in situ* hybridization assays), PCR methods (polymerase chain assays), LCR methods (ligase chain reactions), SD methods (strand displacement assays), competitive hybridization, and the like. In these methods, after addition of a nucleic acid probe labeled with a fluorescent dye, the following operations are performed. (i) The fluorescent dye in the unreacted probe (i.e., probe not hybridized with a target nucleic acid) is removed from the measuring system by washing or the like. (ii) The fluorescent dye, with which the nucleic acid probe hybridized with the target nucleic acid is labeled, is allowed to emit fluorescence from the probe either directly or by applying an indirect measure to the probe (for example, causing an enzyme to act). Then, (iii) the amount of emission is measured. The present invention is characterized by measuring the nucleic acid of interest without performing these complicated operations.

The term "target nucleic acid" as used herein means a nucleic acid the quantification or quantitative detection or mere detection of which is intended, irrespective of whether it is in a purified form or not and further irrespective of its concentration. Various other nucleic acids may also exist together with the target nucleic acid. For example, the target nucleic acid may be a specific nucleic acid in a co-cultivation system of multiple microorganisms (a mixed system of RNAs or genetic DNAs from plural microorganisms) or a symbiotic cultivation system of microorganisms (a mixed system of RNAs or genetic DNAs from plural animals, plants and/or plural microorganisms), the quantitation or quantitative detection or mere detection of which is intended. Purification of the target nucleic acid, if necessary, can be conducted by conventional known methods. For example, purification can be conducted using a commercial purification kit or the like. Specific examples of the above-described nucleic acid include, but are not limited to, DNAs, RNAs, PNAs, 2-O-methyl (Me) RNAs, deoxyribo-oligonucleotides and riboxy-oligonucleotides.

In the present invention, fluorescent dyes commonly employed to label nucleic acid probes for measurement/detection of nucleic acids may be conveniently used. Preferably, fluorescent dyes are used whose emission decreases when a nucleic acid probe labeled with the fluorescent dye is hybridized to a target nucleic acid. Examples of such fluorescent dyes include, but are not limited to, fluorescein and derivatives thereof [e.g., fluorescein isothiocyanate (FITC) or derivatives thereof, Alexa 488, Alexa 532, cy3, cy5, 6-joe, EDANS (5-(2'-aminoethyl)amino-1-naphthalene sulfonic acid)], rhodamine 6G (R6G) or derivatives thereof [e.g., tetramethylrhodamine (TMR), tetramethylrhodamine isothiocyanate (TMRITC), x-rhodamine], Texas red, BODIPY™ FL (product of Molecular Probes/Thermo Fisher Scientific, U.S.A.), BODIPY™ FL/C3 (Molecular Probes/Thermo Fisher Scientific, U.S.A.), BODIPY™ FL/C6 (Molecular Probes/Thermo Fisher Scientific, U.S.A.), BODIPY™ 5-FAM (Molecular Probes/Thermo Fisher Scientific, U.S.A.), BODIPY™ TMR (Molecular Probes/Thermo Fisher Scientific, U.S.A.) or derivatives thereof [e.g., BODIPY™ TR (Molecular Probes/Thermo Fisher Scientific, U.S.A.), BODIPY™ R6G (Molecular Probes/Thermo Fisher Scientific, U.S.A.), BODIPY™ 564 (Molecular Probes/Thermo Fisher Scientific, U.S.A.), BODIPY™ 581 (Molecular Probes/Thermo Fisher Scientific, U.S.A.)], Pacific Blue (Cat. No. P10163; Thermo Fisher Scientific), ATTO465 (Cat. No. AD 465-31; ATTO-TEC), 5-CR 6G (Cat. No. C6127; Thermo Fisher Scientific), 6-TAMRA (Cat. No. C6123; Thermo Fisher Scientific), ATTO655 (Cat. No. AD 655-31; ATTO-TEC), ATTO680 (Cat. No. AD 680-31; ATTO-TEC) and ATTO700 (Cat. No. AD 700-31; ATTO-TEC). Among these fluorescent dyes, FITC, EDANS, 6-joe, TMR, Alexa 488, Alexa 532, BODIPY™ FL/C3 (Molecular Probes/Thermo Fisher Scientific, U.S.A.), BODIPY™ FL/C6 (Molecular Probes/Thermo Fisher Scientific, U.S.A.), Pacific Blue (Cat. No. P10163; Thermo Fisher Scientific), ATTO465 (Cat. No. AD 465-31; ATTO-TEC), 5-CR 6G (Cat. No. C6127; Thermo Fisher Scientific), 6-TAMRA (Cat. No. C6123; Thermo Fisher Scientific), ATTO655 (Cat. No. AD 655-31; ATTO-TEC), ATTO680 (Cat. No. AD 680-31; ATTO-TEC) and ATTO700 (Cat. No. AD 700-31; ATTO-TEC) are preferred, with FITC, TMR, 6-joe, BODIPY™ FL/C3 (Molecular Probes/Thermo Fisher Scientific, U.S.A.), BODIPY™ FL/C6 (Molecular Probes/Thermo Fisher Scientific, U.S.A.), Pacific Blue (Cat. No. P10163; Thermo Fisher Scientific), ATTO465 (Cat. No. AD 465-31; ATTO-TEC), 5-CR 6G (Cat. No. C6127; Thermo Fisher Scientific), 6-TAMRA (Cat. No. C6123; Thermo Fisher Scientific), ATTO655 (Cat. No. AD 655-31; ATTO-TEC), ATTO680 (Cat. No. AD 680-31; ATTO-TEC) and ATTO700 (Cat. No. AD 700-31; ATTO-TEC) being more preferred.

The nucleic acid probe of the invention to be hybridized with the target nucleic acid may be composed of either

an oligodeoxyribonucleotide or an oligoribonucleotide. Alternatively, the nucleic acid probe may be composed of a chimeric oligonucleotide comprising both an oligodeoxyribonucleotide and an oligoribonucleotide. It is also possible to use a 2'-o-methyl oligoribonucleotide in which the nucleoside portion at the 5' end of the oligoribonucleotide is cytidine and the OH group at position 2' of the cytidine is modified by a methyl group. Alternatively, to enhance affinity for RNA, the above-described 2'-o-methyl oligoribonucleotide may be inserted in an oligodeoxynuclueotide. If desired, an artificial, NO binding, bridged nucleic acid (2',4'-BNANC) may also be used.

The number of bases in the probe of the present invention is 5 to 100, preferably 10 to 30 and most preferably 15 to 25. If the number of bases exceeds 100, synthesis errors tend to occur, increasing the chance for the inclusion of probes having sequences other than those of interest. This would narrow the scope of application of the present invention. If the number of bases is less than 5, non-specific hybridization tends to be evoked, leading to increased errors in measurement.

The base sequence of the probe is not particularly limited as long as it specifically hybridizes with the target nucleotide acid. Preferably, the base sequence is so designed that, when the nucleic acid probe labeled with the fluorescent dye is hybridized with the target nucleic acid, (1) at least one G (guanine) exists in the base sequence of the target nucleic acid at a position 1 to 3 bases apart from a terminal base portion of the target nucleic acid hybridizing with the probe; or (2) base pairs in a probe-nucleic acid hybrid complex form at least one G (guanine) and C (cytosine) pair at the terminal site of the probe.

The oligonucleotide in the nucleic acid probe of the present invention can be produced by conventional production methods for general oligonucleotides. It can be produced by, for example, a chemical synthesis method or a microbial method which uses a plasmid vector, a phage vector or the like (Tetrahedron Letters, Vol. 22, pp. 1859-1862, 1981; Nucleic Acids Research, Vol. 14, pp. 6227-6245, 1986). Note that it is suitable to use a nucleic acid synthesizer currently available on the market (e.g., model ABI394, Perkin Elmer, USA).

For labeling the oligonucleotide with a fluorescent dye, any of conventionally known labeling methods may be used as desired (Nature Biotechnology, Vol. 14, pp. 303-308, 1996; Applied and Environmental Microbiology, Vol 63, pp. 1143-114, 1997; Nucleic Acids Research, Vol 24, pp. 4532-4535, 1996). For example, when a fluorescent dye molecule is intended to be bound to the 5' end of the oligonucleotide, an ssH amino linker is first introduced to the phosphate group at the 5' end according to the method disclosed in Japanese Patent No. 4336820. Then, a fluorescent dye having reactivity with amino groups or a derivative thereof may be bound to this linker, whereby a labeled oligonucleotide can be synthesized. The thus synthesized oligonucleotide which is labeled with the fluorescent dye may be purified by chromatography (e.g., reversed-phase) or the like to provide a nucleic acid probe for use in the present invention.

Alternatively, a fluorescent dye may be bound to the 3' end of the oligonucleotide. For this purpose, a CA amino linker is introduced onto an OH group on the C atom at the 3' position of ribose or deoxyribose; or a phosphate group is introduced and then a CA amino linker is introduced onto the OH group of the phosphate group. Then, a fluorescent dye having reactivity with amino groups or a derivative thereof may be bound to this linker, whereby a labeled oligonucleotide can be synthesized. The thus synthesized oligonucleotide which is labeled with the fluorescent dye may be purified by chromatography (e.g., reversed-phase) or the like to provide a nucleic acid probe for use in the present invention. For the introduction of the amino group, a fluorescent dye molecule may be bound to the oligoribonucleotide according to the method disclosed in Japanese Patent No. 4336820. Alternatively, it is also possible to introduce a fluorescent dye molecule into the strands of the nucleic acid probe (Analytical Biochemistry 225, pp. 32-38, 1998). The nucleic acid probe of the present invention can be prepared as described above. A preferred probe form is one labeled with a fluorescent dye at the 3' or 5' end, the labeled terminal base being G or C. If the 5' end is labeled and the 3' end is not labeled, the OH group on the C atom at the 3' position of the 3' end ribose or deoxyribose may be modified with a phosphate group or the like; or the OH group on the C atom at the 2' position of the 3' end ribose may be modified with a phosphate group or the like. No limitation is imposed in this respect.

Use of the nucleic acid probe of the present invention is not limited to measurement of nucleic acids; it can also be suitably applied in methods for analyzing or measuring polymorphisms or/and mutations of target nucleic acids or genes. In particular, the nucleic acid probe of the present invention provides a more convenient method when used in combination with a DNA chip to be described below. Stated briefly, the intensity of fluorescence upon hybridization of the nucleic acid probe of the present invention with a target nucleic acid or gene changes depending on whether a GC pair is formed or not. It is, therefore, possible to analyze or measure polymorphism or/and mutation of a target nucleic acid or gene by hybridizing the nucleic acid probe of the present invention with the target nucleic acid or gene and then measuring the intensity of emission. In this analysis or measurement, the target nucleic acid or gene may be either an amplified product, as obtained by one of the various nucleic acid or gene amplification methods, or an extract of such amplified product. Further, the target nucleic acid may be of any type. Examples of the target nucleic acid to which the present invention is applicable include, but are not limited to, RNAs, DNAs, PNAs, 2',4'-BNA, 2',4'-BNA$^{COC}$, 3'-amino-2',4'-BNA, 2',4'-BNA$^{NC}$, and other artificially modified nucleic acids. It should be noted here that the only requisite for target nucleic acids is that a guanine base be present within the strand(s) or at the end(s). If no guanine base is present

within the strand(s) or at the end(s), the intensity of fluorescence will not decrease.

The nucleic acid probe of the present invention may be contained in a measurement kit for analyzing or measuring polymorphisms and mutations of a target nucleic acid or/and gene and the kit can suitably be used for analyzing or measuring polymorphisms and mutations of the target nucleic acid or/and gene.

In the present invention, use of the above-described nucleic acid probe makes it possible to specifically measure a target nucleotide with convenience in a short time. Hereinbelow, the measuring method will be described. In the measuring method of the present invention, the above-described nucleic probe is first added to a measuring system and allowed to hybridize with a target nucleic acid. This hybridization may be performed by usual, known methods (Analytical Biochemistry, Vol. 183, pp. 231-244, 1989; Nature Biotechnology, Vol 14, pp. 303-308, 1996; Applied and Environmental Microbiology, Vol. 63, pp. 1143-114, 1997). As conditions for hybridization, the salt concentration may, for example, range from 0 to 2 molar concentration, preferably from 0.1 to 1.0 molar concentration, with pH from 6 to 8, preferably from 6.5 to 7.5.

The reaction temperature is preferably within a range of Tm±10°C, Tm being the value for the hybrid complex which is to be obtained by hybridization of the nucleic acid probe to the specific site of the target nucleic acid. By setting the reaction temperature within such a range, non-specific hybridization can be prevented. If the reaction temperature is less than Tm-10°C, non-specific hybridization occurs; if the reaction temperature is more than Tm+10°C, hybridization does not occur. It should be noted that the Tm value can be determined in the same manner as in an experiment which is needed to design the nucleic acid probe for use in the present invention. Stated briefly, an oligonucleotide which is to hybridize with the nucleic acid probe and has a complementary base sequence to the nucleic acid probe is chemically synthesized with the above-mentioned nucleic acid synthesizer, and the Tm value of the hybrid complex between the oligonucleotide and the nucleic acid probe is then measured by conventional methods.

Further, the reaction time may range from 1 sec to 180 min, preferably from 5 sec to 90 min. If the reaction time is less than 1 sec, a substantial portion of the nucleic acid probe of the present invention will remain unreacted in the hybridization. On the other hand, no particular advantage can be brought about even if the reaction time is set excessively long. The reaction time is greatly affected by the type of the nucleic acid (i.e., the length or base sequence of the nucleic acid). In the present invention, the nucleic acid probe is hybridized with the target nucleic acid as described above. The intensity of emission from the fluorescent dye is measured with a fluorometer both before and after the hybridization, and the amount of decrease in emission is calculated. Since the magnitude of the decrease is proportional to the amount of the target nucleic acid, it is possible to determine the amount of the target nucleic acid.

The concentration of the target nucleic acid in the reaction solution preferably ranges from 0.1 to 10.0 nM. The concentration of the probe in the reaction mixture preferably ranges from 1.0 to 25.0 nM. For preparing a calibration curve, the probe may desirably be used at ratios of from 1.0 to 2.5 relative to the target nucleic acid.

For actually measuring a target nucleic acid of unknown concentration in a sample, a calibration curve is first prepared under the above-described conditions. Then, a corresponding probe is added at a plurality of concentrations to aliquots of the sample, respectively, followed by measurement of decreases in fluorescence intensity. Subsequently, the probe concentration at which the measured decrease in fluorescence intensity is at maximum is chosen as a preferred probe concentration. Based on the decrease in fluorescence intensity as measured with the probe of the preferred concentration, a quantified value of the target nucleic acid can be determined from the calibration curve.

While the principle of the measuring method of the present invention can be applied to any method insofar as it is a PCR method, a description will hereinafter be made of the case where it is applied to a real-time quantitative PCR method. Stated briefly, in the real-time quantitative PCR method, PCR is performed using a specific nucleic acid probe of the present invention, and a decrease in the emission from the fluorescent dye before a reaction relative to the emission from the fluorescent dye after the reaction is measured in real time. The term "PCR" as used herein means a variety of PCR methods. Examples can include RT-PCR, RNA-primed PCR, stretch PCR, reverse PCR, PCR making use of Alu sequence(s), multiple PCR, PCR using mixed primers, and PCR using PNA. Further, the term "quantitative" as used herein shall mean not only quantification in the proper sense but also quantification of an accuracy that is virtually equivalent to detection.

As described above, the "target nucleic acid" means a nucleic acid the quantification or quantitative detection or mere detection of which is intended irrespective of whether it is in a purified form or not and further irrespective of its concentration. Various other nucleic acids may also exist together with the target nucleic acid. For example, the target nucleic acid may be a specific nucleic acid to be amplified in a co-cultivation system of microorganisms (a mixed system of RNAs or genetic DNAs from a plurality of microorganisms) or a symbiotic cultivation system of microorganisms (a mixed system of RNAs or genetic DNAs from a plurality of animals, plants and/or microorganisms). Purification of the target nucleic acid, if necessary, can be performed by conventionally known methods. For instance, it can be performed using a commercial purification kit or the like.

The conventionally known quantitative PCR methods is such that a target nucleic acid (DNA or RNA) is amplified in the presence of Mg ions using dATP, dGTP, dCTP, dTTP or dUTP, the target nucleic acid, Taq polymerase, primers and a nucleic acid probe labeled with a fluorescent dye or an intercalator while repeatedly changing the temperature

between low and high levels, and that the increases in fluorescence emission from the fluorescent dye in the course of the amplification are monitored in real time [Jikken Igaku (Laboratory Medicine), 15(7), 46-51, Yodosha (1997)].

The quantitative PCR method of the present invention is characterized in that the target nucleic acid is amplified using the nucleic acid probe of the present invention and that the amount of decrease in the emission from the fluorescent dye is measured in the course of amplification. For use in the quantitative PCR method of the present invention, a preferred probe of the present invention may comprise bases ranging in number from 5 to 100, preferably from 10 to 30, and especially preferably from 15 to 25. Any probe will do as long as it hybridizes with the amplified product of the target nucleic acid during PCR cycles. The probe may be designed as either a forward type or a reverse type.

As examples of preferred nucleic acid probes, the following may be enumerated.

(1) A nucleic acid probe labeled, at a 5' end portion, preferably the 5' end, thereof with a fluorescent dye useful for carrying out the present invention, wherein the base sequence thereof is so designed that, when hybridized at the end portion with a target nucleic acid, at least one G (guanine) base exist in the base sequence of the target nucleic acid at a position 1 to 3 bases apart toward the 5' side from the base on the 5' end at which the probe and the target nucleic acid are hybridized with each other.

(2) The probe of (1) above, wherein the 3' end is labeled with the fluorescent dye.

(3) The probe of (1) above, wherein the 3' end (e.g., the OH group on the C atom at the 3' position of ribose or deoxyribose at the 3' end, or the OH group on the C atom at the 2' position of ribose at the 3' end) is modified with a phosphate group or the like.

(4) A nucleic acid probe labeled, at a 3' end portion, preferably the 3' end, thereof with the fluorescent dye of the present invention, wherein the base at the 3' end is G or C, or the OH group on the C atom at the 2' position of ribose at the 3' end is modified with a phosphate group or the like.

(5) The probe of (1) above, wherein the 5' end portion, preferably the 5' end, is labeled with the fluorescent dye of the present invention.

(6) A nucleic acid probe labeled, at a 5' end portion, preferably the 5' end, thereof with a fluorescent dye useful for carrying out the present invention, wherein the base at the 5' end is GorC.

In each of the probes (4) and (6) above, it may not be possible by any means to design the 5' end as G or C due to the base sequence of the relevant target nucleic acid. If this should be the case, 5'-guanylic acid or 5'-cytidylic acid may be added to the 5' end of an oligonucleotide designed as a primer from the base sequence of the target nucleic acid. The probe so obtained can still achieve the object of the present invention. The expression "nucleic acid probe designed so that the base at the 3' or 5' end becomes G or C" as used herein is, therefore, defined to encompass not only probes designed from the base sequence of the target nucleic acid but also probes modified therefrom by adding 5'-guanylic acid or 5'-cytidylic acid to the 3' or 5' end.

In particular, the above-described probe (1), (2), (3) or (4) is designed so that it will not be used as a primer. Instead of two (fluorescently labeled) probes used in a real-time quantitative PCR method making use of the FRET phenomenon, a single probe of the present invention is used to perform PCR. The probe is added to a PCR reaction system and PCR is then performed. During a nucleic acid extending reaction, the probe which has hybridized with the target nucleic acid or amplified target nucleic acid is degraded by polymerase to be eliminated from the hybrid complex. The intensity of fluorescence from the reaction system at this time or from the reaction system in which a nucleic acid denaturing reaction has completed is measured. Further, the intensity of fluorescence from the reaction system in which the target nucleic acid or amplified target nucleic acid has hybridized with the probe (i.e., the reaction system at the time of an annealing reaction or at the time of the nucleic acid extending reaction until the probe is removed from the hybrid complex by polymerase) is measured. By calculating a decrease of fluorescence intensity in the latter case from the value in the former case, the concentration of the amplified nucleic acid is measured. The intensity of fluorescence is high when the probe has been completely dissociated from the target nucleic acid or amplified target nucleic acid by the nucleic acid denaturing reaction, or when the probe has been removed from the hybrid complex of the probe and the target nucleic acid or amplified target nucleic acid by polymerase during extension of the nucleic acid. However, the intensity of fluorescence from the reaction system in which an annealing reaction has been completed and the probe has fully hybridized to the target nucleic acid or amplified target nucleic acid, or from the reaction system until the probe has been removed from the hybrid complex of the probe and the target nucleic acid or amplified target nucleic acid upon degradation by polymerase at the time of a nucleic acid extending reaction is lower than the value in the former case. The decrease in the intensity of fluorescence is proportional to the amount of the amplified nucleic acid. In this case, each of the base sequences of the probes (2), (3) and (4) are desirably designed so that the Tm of a hybrid complex, which is available upon hybridization of the probe with the target nucleic acid, comes within the range of the Tm value of a primer's hybrid complex ±15°C, preferably ±5°C. If the Tm value of the probe is less than the primer's Tm value minus 5°C, especially minus 15°C, the probe does not hybridize, so there will occur no decrease in the emission from the fluorescent dye. If, on the other hand, the Tm value of the probe is above the primer's Tm plus 5°C, especially plus

15°C, the probe also hybridizes to nucleic acids other than the target nucleic acid, so the specificity of the probe is lost.

The probes (5) and (6) above are added as primers to the PCR reaction system. Except for the PCR method of the present invention, no PCR method is known to make use of a primer labeled with a fluorescent dye. As the PCR reaction proceeds, the amplified nucleic acid is progressively labeled with the fluorescent dye of the present invention. Accordingly, the intensity of fluorescence from the reaction system in which the nucleic acid denaturing reaction has completed is high but, in the reaction system in which the annealing reaction has completed or the nucleic acid extending reaction is in progress, the intensity of fluorescence is lower than the value in the above-described former case.

The PCR reaction may be carried out under the same reaction conditions as in conventional PCR methods. It is therefore possible to perform amplification of a target nucleic acid in a reaction system containing Mg ions at low concentrations (1 to 2 mM). Needless to say, the present invention may also be carried out even in a reaction system containing Mg ions at high concentrations (2 to 4 mM) that are employed in the conventionally known quantitative PCR.

In the PCR method of the present invention, Tm values can be determined by performing the PCR of the present invention and then analyzing the melting curve of the nucleic acid with respect to the resultant amplification products. This method is a novel method for analyzing a melting curve of a nucleic acid. In this method, the nucleic acid probe used in the PCR method of the present invention or the nucleic acid probe used as a primer may suitably be used. In this case, the probe of the present invention may be designed to comprise a base sequence complementary to a region containing an SNP (single nucleotide polymorphism) in the target nucleic acid, so that after completion of PCR, a dissociation curve of the target nucleic acid from the probe of the present invention is analyzed, whereby the SNP can be detected from a difference, if any, in the dissociation curve. If a base sequence complementary to an SNP-containing sequence is used as a sequence for the probe of the present invention, a Tm value available from a dissociation curve between the sequence of the probe and the SNP-containing sequence becomes higher than a Tm value available from a dissociation curve between the sequence of the probe and the SNP-free sequence.

## EXAMPLES

Hereinbelow, the present invention will be described in more detail with reference to the following Examples.

[Example 1]

### Experimental Summary

A plurality of QProbes with different alkyl chain lengths were synthesized by using conventional amino linkers with different lengths of alkyl chains for linking an oligo DNA and a fluorescent dye, and the fluorescent dye BODIPY™ FL. Then, the effect of alkyl chain length upon the fluorescence quenching rates of QProbes was investigated. In Fig. 4, the structure of the QProbes prepared in this Example is shown. Using three types of amino linkers with different alkyl chain lengths (carbon number in the alkyl chain is 3, 6 or 12) and two types of fluorescent dye BODIPY™ FL with different alkyl chain lengths (carbon number in the alkyl chain is 2 or 4), a total of six QProbes were synthesized which had different lengths of alkyl chains for linking an oligo DNA and a fluorescent dye. It should be noted here that all of the six QProbes had a common sequence and C (cytosine) at the 5' end was fluorescently labeled with BODIPY™ FL.

Table 1. Summary of QProbes Used for Investigation

| No. | Existing amino linker | | | Fluorescent dye: BODIPY™ FL | | | Total alkyl chain length |
|---|---|---|---|---|---|---|---|
| | Manufacturer | Alkyl chain length | Cat. No. | Manufacturer | Alkyl chain length | Cat. No. | |
| 1 | Glen Research | C3 | 10-1923-90 | Thermo Fisher Scientific | C2 | D6140 | C5 |
| 2 | ditto | C3 | 10-1923-90 | ditto | C4 | D6184 | C7 |
| 3 | ditto | C6 | 10-1906-90 | ditto | C2 | D6140 | C8 |
| 4 | ditto | C6 | 10-1906-90 | ditto | C4 | D6184 | C10 |
| 5 | ditto | C12 | 10-1912-90 | ditto | C2 | D6140 | C14 |

(continued)

| No. | Existing amino linker | | | Fluorescent dye: BODIPY™ FL | | | Total alkyl chain length |
|---|---|---|---|---|---|---|---|
| | Manufacturer | Alkyl chain length | Cat. No. | Manufacturer | Alkyl chain length | Cat. No. | |
| 6 | ditto | C12 | 10-1912-90 | ditto | C4 | D6184 | C16 |
| QProbe sequence (5' →3'): CCTACGGGAGGCAGCAG (SEQ ID NO: 1)<br>Complementary strand sequence: (5' →3'): CTGCTGCCTCCCGTAGG (SEQ ID NO: 2)<br>Fluorescently labeled site: Cytosine at the 5' end | | | | | | | |

Table 2. Volume and Composition of Reaction Mixture in Example 1

| | System to which both QProbe and complementary strand were added | System to which QProbe alone was added |
|---|---|---|
| Total Volume: | 20 μl | same as on the left |
| QProbe: | 50 nM | same as on the left |
| Complementary strand: | 1,600 nM | 0 nM (not added) |
| KCl: | 50mM | same as on the left |
| Tris-HCl: | 10mM (pH 8.7 at 25°C) | same as on the left |
| $MgCl_2$ : | 1.5mM | same as on the left |

For each of the six QProbes, two reaction systems were provided; to one reaction system, both QProbe and complementary strand were added, and to the other reaction system, QProbe alone was added. Dissociation curve analysis was performed using a real-time PCR instrument (LightCycler™ 480; Roche Life Science). Fluorescence measurements in the dissociation curve analysis were performed within a temperature range of 40 to 95°C, and the frequency of fluorescence measurements was approx. 0.2°C/measurement. Using the thus obtained fluorescence intensity data, fluorescence quenching rate at each measurement temperature was determined from the calculation formula described below. The highest value obtained was taken as maximum fluorescence quenching rate, and the measured values were compared among the six QProbes.

$$\text{Fluorescence quenching rate (\%)}=\{(F1-[F2\times F1_{95}/F2_{95}])\ /F1\}\times 100$$

wherein

F1: fluorescence intensity of [QProbe alone]
F2: fluorescence intensity of [QProbe + complementary strand]
$F1_{95}$: fluorescence intensity of [QProbe alone] at 95°C (at the time of dissociation)
$F2_{95}$: fluorescence intensity of [QProbe + complementary strand] at 95°C (at the time of dissociation)

The results are shown in Fig. 5. From these results, it is understood that fluorescence quenching was maximum (about 82%) when the alkyl chain length was 8 in terms of carbon number (C8). When C7 QProbe (shorter than C8 QProbe by one carbon atom) was used, the fluorescence quenching rate was about 59%, a substantial decrease from the corresponding rate of C8 QProbe. On the other hand, in the case of C10 QProbe longer than C8 QProbe by two carbon atoms, fluorescence quenching rate was about 54%, again a substantial decrease from the corresponding rate of C8 QProbe.

These results show that a small difference in alkyl chain length greatly affects the fluorescence quenching rate of QProbe. Since the sensitivity and accuracy in the detection of a target gene using a QProbe largely depend on the fluorescence quenching rate, these results also suggest that selection of an alkyl chain is an important factor that determines the performance of QProbes.

[Example 2]

The results of Example 1 have shown that the structure of the alkyl chain for linking an oligo DNA and a fluorescent dye largely affects the fluorescence quenching rate which is directly connected to the performance of QProbes.

In this connection, when the chemical structures of QProbes synthesized with existing amino linkers and QProbes synthesized with novel amino linkers are compared (see Fig. 3), it is understood that the latter linkers are longer, providing a greater distance between an oligonucleotide and a fluorescent dye. Considering this point together with the results of Example 1, a comparison between the fluorescence quenching rate of a QProbe synthesized with an optimal existing amino linker (C6 amino linker in the case of BODIPY™ FL [D6140]) and the fluorescence quenching rate of a QProbe synthesized with a novel amino linker with the same carbon number led the present inventors to anticipate that the fluorescence quenching rate of the latter might be worsened significantly.

In order to verify this possibility, the present inventors provided QProbes as synthesized with an optimal existing amino linker (consignee: Tsukuba Oligo Service) and QProbes as synthesized with a novel amino linker having the same alkyl chain length as the optimal existing amino linker (consignee: GeneDesign, Inc.) and compared their maximum fluorescence quenching rates of these QProbes.

Table 3-1. Summary of QProbes Used in Example 2 (fluorescently labeled at 5' end)

| Designation of QProbe | Fluorescent Dye | Amino Linker |
|---|---|---|
| PB-5'-S | Pacific Blue | Existing amino linker |
| PB-5'-N | Ditto | Novel amino linker |
| A46-5'-S | ATTO 465 | Existing amino linker |
| A46-5'-N | Ditto | Novel amino linker |
| BF-5'-S | BODIPY FL | Existing amino linker |
| BF-5'-N | Ditto | Novel amino linker |
| CR-5'-S | 5-CR 6G | Existing amino linker |
| CR-5'-N | Ditto | Novel amino linker |
| TR-5'-S | 6-TAMRA | Existing amino linker |
| TR-5'-N | Ditto | Novel amino linker |
| A65-5'-S | ATTO 655 | Existing amino linker |
| A65-5'-N | Ditto | Novel amino linker |
| A68-5'-S | ATTO 680 | Existing amino linker |
| A68-5'-N | Ditto | Novel amino linker |
| A70-5'-S | ATTO 700 | Existing amino linker |
| A70-5'-N | Ditto | Novel amino linker |

Table 3-2. Summary of QProbes Used in Example 2 (sequence fluorescently labeled at 3' end (5' →3'))

| Designation of QProbe | Fluorescent Dye | Amino Linker |
|---|---|---|
| PB-3'-S | Pacific Blue | Existing amino linker |
| PB-3'-N | Ditto | Novel amino linker |
| A46-3'-S | ATTO 465 | Existing amino linker |
| A46-3'-N | Ditto | Novel amino linker |
| BF-3'-S | BODIPY FL | Existing amino linker |
| BF-3'-N | Ditto | Novel amino linker |
| CR-3'-S | 5-CR 6G | Existing amino linker |
| CR-3'-N | Ditto | Novel amino linker |

(continued)

| Designation of QProbe | Fluorescent Dye | Amino Linker |
|---|---|---|
| TR-3'-S | 6-TAMRA | Existing amino linker |
| TR-3'-N | Ditto | Novel amino linker |
| A65-3'-S | ATTO 655 | Existing amino linker |
| A65-3'-N | Ditto | Novel amino linker |
| A68-3'-S | ATTO 680 | Existing amino linker |
| A68-3'-N | Ditto | Novel amino linker |
| A70-3'-S | ATTO 700 | Existing amino linker |
| A70-3'-N | Ditto | Novel amino linker |

<Additional Explanation for Table 3>

Sequence of QProbe: QProbe fluorescently labeled at the 5' end: 5' CCTACGGGAGGCAGCAG 3' (SEQ ID NO: 1)
QProbe fluorescently labeled at the 3' end: 5' AAGGAGGTGATCCAGCC 3' (SEQ ID NO: 3)
Designation of QProbe: expressed as ○○○-Δ'-□

○○○: abbreviation of fluorescent dye
Δ': fluorescently labeled end. Therefore, Δ reads "5" or "3".
□: linker used. "S" indicates the use of an existing amino linker and "N" a novel amino linker.

Length of linker's alkyl chain: Carbon number 6 (common to all QProbes used in Example 2)
Existing amino linker: 5' end: 5'-Amino-Modifier C6 (Cat. No. 10-1906-90; Glen Research)
3' end: 3'-PT-Amino-Modifier C6 CPG (Cat. No. 20-2956-01; Glen Research)
Novel amino linker: 5' end: ssH Amino Linker (Cat. No. CLP-1132; ChemGenes)
3' end: 3'-Amino CA Linker (synthesis of QProbes using this linker was consigned to GeneDesign, Inc.)
Fluorescent dye: Pacific Blue (Cat. No. P10163; Thermo Fisher Scientific)

ATTO465 (Cat. No. AD 465-31; ATTO-TEC)
BODIPY™ FL (Cat. No. D6140; Thermo Fisher Scientific)
5-CR 6G (Cat. No. C6127; Thermo Fisher Scientific)
6-TAMRA (Cat. No. C6123; Thermo Fisher Scientific)
ATTO655 (Cat. No. AD 655-31; ATTO-TEC)
ATTO680 (Cat. No. AD 680-31; ATTO-TEC)
ATTO700 (Cat. No. AD 700-31; ATTO-TEC)

Complementary strand: Oligo DNA having a sequence which is of the same strand length as and completely complementary to QProbe (synthesized by Tsukuba Oligo Service)

In this Example, 16 QProbes using existing amino linkers were provided, and the same number of QProbes were provided in the same way, except that in each of them, the linker alone was changed to a novel amino linker.

Maximum fluorescence quenching rate was determined for a total of the 32 QProbes mentioned above in the same manner as described in Example 1. Then, the effect of novel amino linkers on fluorescence quenching rate was investigated by comparing the measured values.

The results for the QProbes in which cytosine at the 5' end was fluorescently labeled are shown in Fig. 6-1. These results showed that, for any of the fluorescent dyes used in labeling, the fluorescence quenching rates of the QProbes using the novel amino linkers were comparable to or higher than the corresponding rates of the QProbes using the existing linkers.

The results for the QProbes in which cytosine at the 3' end was fluorescently labeled are shown in Fig. 6-2. Like the results with the 5' terminally labeled QProbes, these results showed that, for any of the fluorescent dyes used in labeling, the fluorescence quenching rates of the QProbes using the novel amino linkers were comparable to or higher

than the corresponding rates of the QProbes using the existing linkers.

Thus, the QProbes using the novel amino linkers have performance that is comparable to or better than that of the QProbes using the conventional amino linkers. Further, as described earlier, a substantial reduction in the production cost is possible. The QProbes using the novel amino linkers were, therefore, shown to have great potential for use in industrial applications.

Example 1 showed that a small difference in linker length greatly affects the fluorescence quenching rate. Since the QProbes synthesized with novel amino linkers have linker lengths that are greater (by at least two carbon atoms) than in the case of the QProbes synthesized with an optimal existing amino linker, it was anticipated that the fluorescence quenching rate of the former QProbes might considerably deteriorate from that of the latter QProbes. However, as described above, the present inventors obtained results demonstrating that the fluorescence quenching rate of the QProbes synthesized with novel amino linkers is comparable to or higher than that of the QProbes synthesized with existing amino linkers. Since these results show an event that is difficult to anticipate without actual verification, the present invention is also recognized to feature an inventive step.

[Example 3]

Comparison of Reactivity with Fluorescent Dye

First, the following four types of aminated oligo DNAs were provided in this Example. Specifically,

[1] aminated oligo DNA in which an existing amino linker has been introduced to the 5' end of the oligo DNA shown in SEQ ID NO: 1,
[2] aminated oligo DNA in which a novel amino linker has been introduced to the 5' end of the oligo DNA shown in SEQ ID NO: 1,
[3] aminated oligo DNA in which an existing amino linker has been introduced to the 3' end of the oligo DNA shown in SEQ ID NO: 3, and
[4] aminated oligo DNA in which a novel amino linker has been introduced to the 5' end of the oligo DNA shown in SEQ ID NO: 3

were provided.

These four types of aminated oligo DNAs were reacted with 8 types of fluorescent dyes usable in QProbe. By measuring/comparing the amounts of the resultant reaction products, difference in reactivity with the above fluorescent dyes was compared between the existing amino linkers and the novel amino linkers. Synthesis of the aminated oligo DNAs having the existing amino linkers introduced therein ([1] and [3] above) was consigned to Tsukuba Oligo Service, and synthesis of the aminated oligo DNAs having the novel amino linkers introduced therein ([2] and [4] above) was consigned to GeneDesign, Inc.

Each of the aminated oligo DNAs [1] to [4] above (1 nmol) and a fluorescent dye (500 nmol) were dissolved in 10% (v/v) dimethylformamide in 250 mM phosphate buffer (pH 8.0) to make a total volume of 100 μl. After shading this solution, reaction of the aminated oligo DNA and the fluorescent dye was started at 40°C. A 15 μl sample was taken 30 min after the start of the reaction and desalted with NAP5 (Pharmacia). Then, the amounts in this sample of the aminated oligo DNA that was unreacted with the fluorescent dye and the aminated oligo DNA that was reacted with the fluorescent dye were determined by reversed phase HPLC.

<Other details of conditions>

Sequence of QProbe: QProbe fluorescently labeled at the 5' end: 5' CCTACGGGAGGCAGCAG 3' (SEQ ID NO: 1)
QProbe fluorescently labeled at the 3' end: 5' AAGGAGGTGATCCAGCC 3' (SEQ ID NO: 3)
Length of linker alkyl chain: Carbon number 6 (common to all QProbes used in Example 3)
Existing amino linker: 5' end: 5'-Amino-Modifier C6 (Cat. No. 10-1906-90; Glen Research)
3' end: 3'-PT-Amino-Modifier C6 CPG (Cat. No. 20-2956-01; Glen Research)
Novel amino linker: 5' end: ssH Amino Linker (Cat. No. CLP-1132; ChemGenes)
3' end: 3'-Amino CA Linker (synthesis of QProbe using this linker was consigned to GeneDesign, Inc.)
Fluorescent dye: Pacific Blue (Cat. No. P10163; Thermo Fisher Scientific)

ATT0465 (Cat. No. AD 465-31; ATTO-TEC)
BODIPY™ FL (Cat. No. D6140; Thermo Fisher Scientific)

5-CR 6G (Cat. No. C6127; Thermo Fisher Scientific)
6-TAMRA (Cat. No. C6123; Thermo Fisher Scientific)
ATT0655 (Cat. No. AD 655-31; ATTO-TEC)
ATTO680 (Cat. No. AD 680-31; ATTO-TEC)
ATTO700 (Cat. No. AD 700-31; ATTO-TEC)
HPLC conditions: Mobile phase: solution A 5% acetonitrile/100 mM TEAA (pH 7.0)
solution B 50% acetonitrile/100 mM TEAA (pH 7.0)

Column temperature: 50°C
Gradient: % of solution B 0 → 70%/30 min

The proportion, as determined by the method described above, of the aminated oligo DNA that reacted with the fluorescent dye is shown in Fig. 7-1 and Fig. 7-2.

Fig. 7-1 is a graph showing the proportion of the aminated oligo DNA that reacted with the fluorescent dye for fluorescent labeling at the 5' end; and Fig. 7-2 is a graph showing the proportion of the aminated oligo DNA that reacted with the fluorescent dye for fluorescent labeling at the 3' end.

These results showed that the proportion of the aminated oligo DNA which reacted with the fluorescent dye was significantly higher in the case of using the novel amino linker than in the case of using the existing amino linker, regardless of the position of fluorescent modification and the type of the fluorescent dye. From these results, it was shown that a large quantity of QProbe can be obtained easily by using the novel amino linker.

Further, from the results of this Example and Example 2, it was shown that a high quality QProbe can be manufactured at a low cost by using the novel amino linker.

All publications, patents and patent applications cited herein are incorporated herein by reference in their entirety.

INDUSTRIAL APPLICABILITY

The present invention is applicable to qualitative and quantitative analysis of target genes.

SEQUENCE LISTING FREE TEXT

<SEQ ID NO: 1>
SEQ ID NO: 1 shows the sequence of a QProbe fluorescently labeled at the 5' end. 5' CCTACGGGAGGCAGCAG 3'
<SEQ ID NO: 2>
SEQ ID NO: 2 shows a strand sequence complementary to the QProbe shown in SEQ ID NO: 1.
5' CTGCTGCCTCCCGTAGG 3'
<SEQ ID NO: 3>
SEQ ID NO: 3 shows the sequence of a QProbe fluorescently labeled at the 3' end.
5' AAGGAGGTGATCCAGCC 3'

## Claims

1. A nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 5' terminal site via an ssH amino linker as shown below, and (iii) the base sequence of the probe is so designed that, when the probe is hybridized with the target nucleic acid, at least one G (guanine) exists in the base sequence of the target nucleic acid at a position 1 to 3 bases apart from the base at the terminal site where the probe and the target nucleic acid are hybridized with each other.

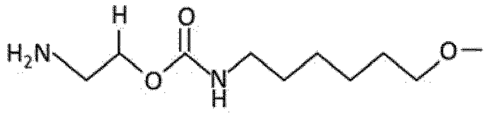

ssH amino linker

2. A nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 3' terminal site via a CA amino linker as shown below, and (iii) the base sequence of the probe is so designed that, when the probe is hybridized with the target nucleic acid, at least one G (guanine) exists in the base sequence of the target nucleic acid at a position 1 to 3 bases apart from the base at the terminal site where the probe and the target nucleic acid are hybridized with each other.

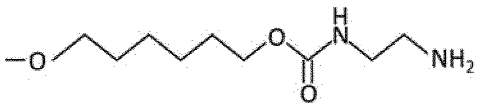

CA amino linker

3. A nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 5' terminal site via an ssH amino linker as shown below, and (iii) the base sequence of the probe is so designed that, when the probe is hybridized with the target nucleic acid, base pairs in a probe-nucleic acid hybrid complex form at least one G (guanine) and C (cytosine) pair at the terminal site.

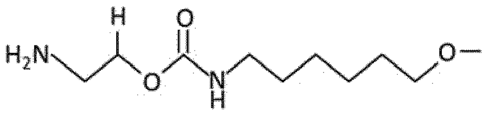

ssH amino linker

4. A nucleic acid probe for measuring a nucleic acid, wherein (i) the probe is labeled with a fluorescent dye whose emission is reduced when the probe is hybridized with a target nucleic acid, (ii) the probe is labeled with the fluorescent dye at its 3' terminal site via a CA amino linker as shown below, and (iii) the base sequence of the probe is so designed that, when the probe is hybridized with the target nucleic acid, base pairs in a probe-nucleic acid hybrid complex form at least one G (guanine) and C (cytosine) pair at the terminal site.

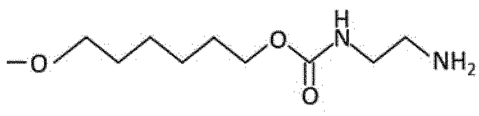

CA amino linker

5. The nucleic acid probe of any one of claims 1 to 4, wherein the terminal base at which the probe is labeled with the fluorescent dye is C (cytosine).

6. The nucleic acid probe of any one of claims 1 to 5, wherein the fluorescent dye is selected from the group consisting of Pacific Blue, ATTO465, BODIPY™ FL, 5-CR 6G, 6-TAMRA, ATTO655, ATTO680 and ATTO700.

7. A method of measuring a nucleic acid using a nucleic acid probe labeled with a fluorescent dye, wherein (i) the probe is labeled with a fluorescent dye whose emission is reduced when the probe is hybridized with a target nucleic acid and (ii) a decrease in the emission from the fluorescent dye is measured, said method being **characterized by** using the nucleic acid probe of any one of claims 1 to 6.

Fig. 1

<Structural formula for the case where an existing linker (3' C6 amino linker) has been introduced to the 3' end of oligo DNA>

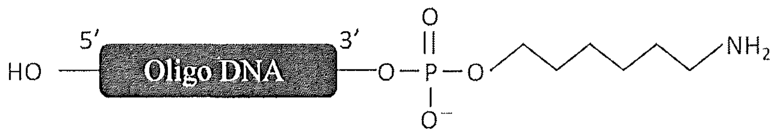

<Structural formula for the case where an existing linker (5' C6 amino linker) has been introduced to the 5' end of oligo DNA>

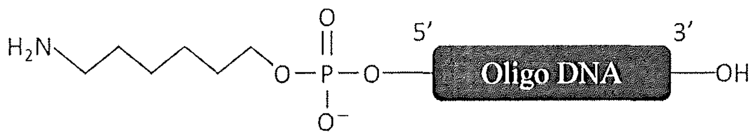

Fig. 2

<Structural formula for the case where a novel amino linker (3' amino CA linker) has been introduced to the 3' end of oligo DNA>

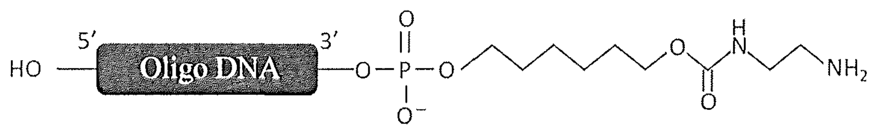

<Structural formula for the case where a novel amino linker (5' SSH amino linker) has been introduced to the 5' end of oligo DNA>

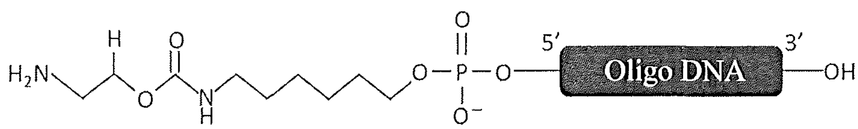

Fig. 3

| | 5' end | 3' end |
|---|---|---|
| Existing amino linker | 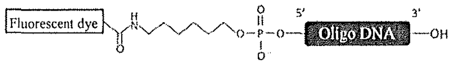 | 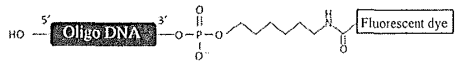 |
| Novel amino linker | 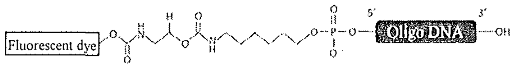 | 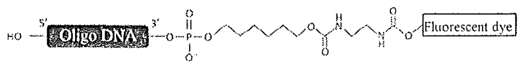 |

Fig. 4

Fluorescent dye side

Linker moiety (C2 or C4)

Amino linker moiety (C3, C6, or C12)

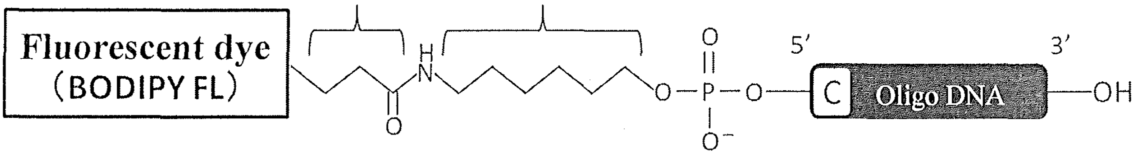

Fig. 5

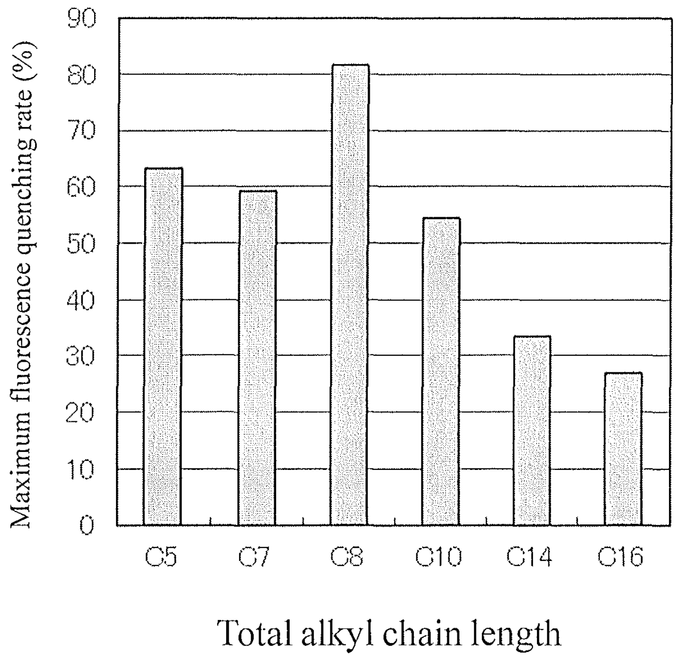

Fig. 6－1

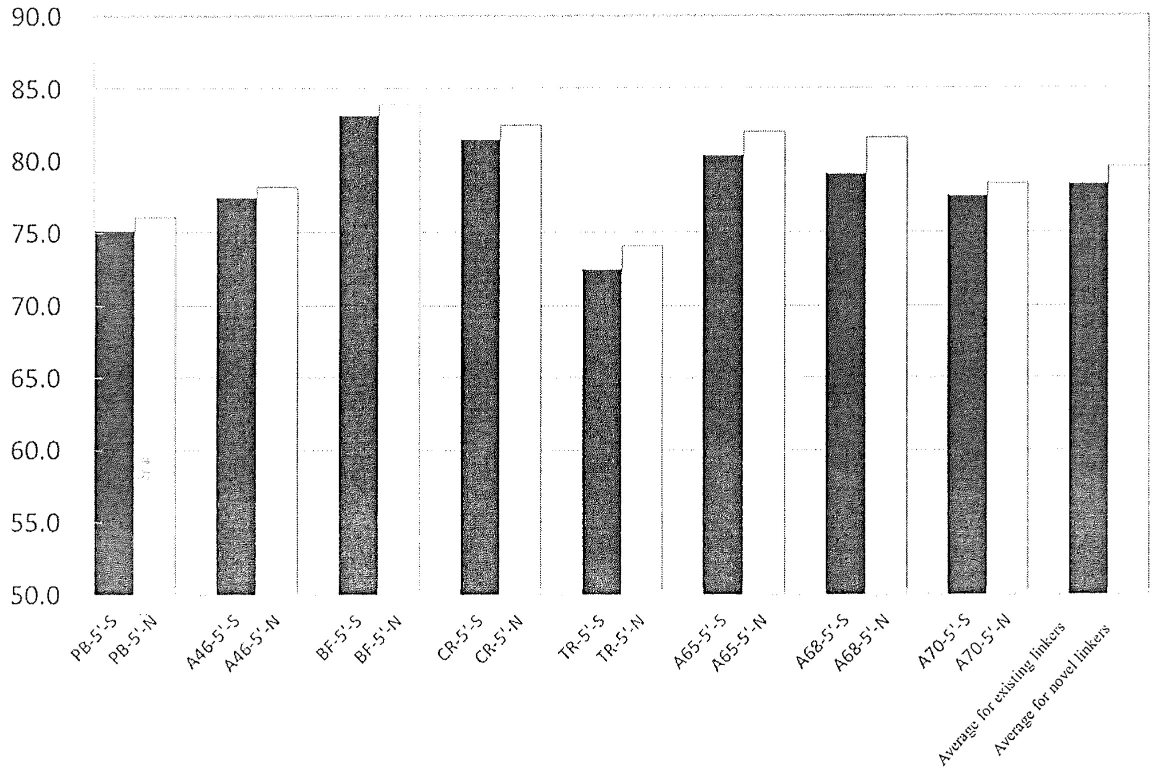
90.0
85.0
80.0
75.0
70.0
65.0
60.0
55.0
50.0
PB-5'-S
PB-5'-N
A46-5'-S
A46-5'-N
BF-5'-S
BF-5'-N
CR-5'-S
CR-5'-N
TR-5'-S
TR-5'-N
A65-5'-S
A65-5'-N
A68-5'-S
A68-5'-N
A70-5'-S
A70-5'-N
Average for existing linkers
Average for novel linkers

Fig. 6－2

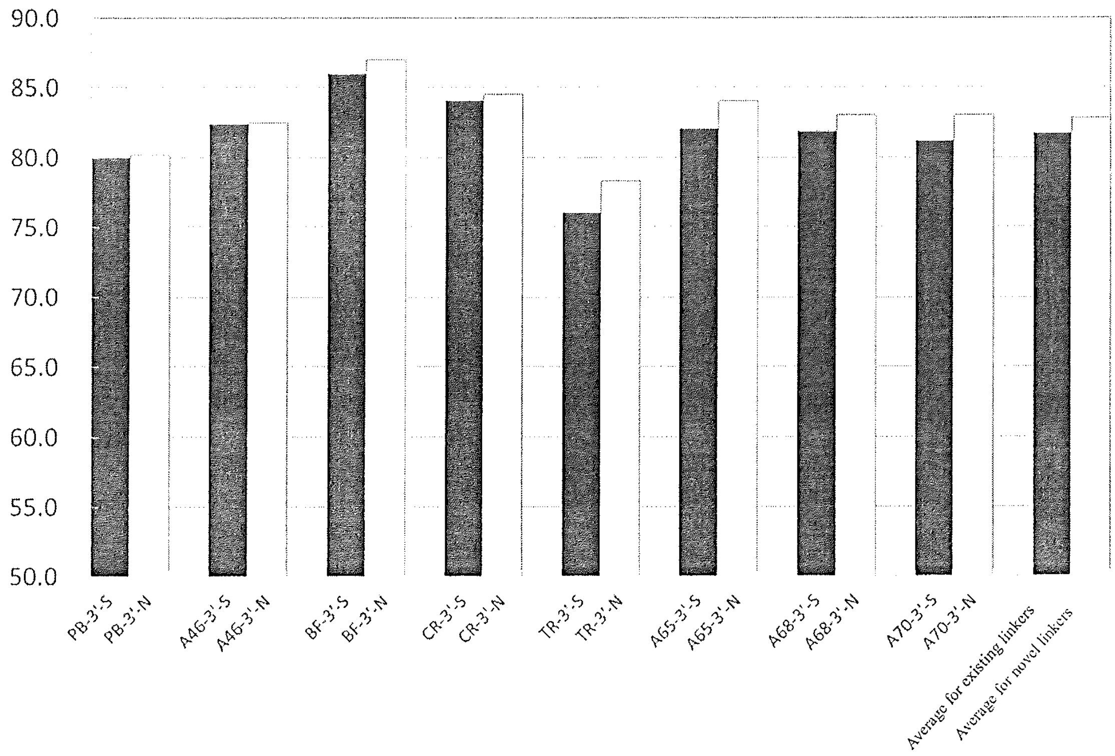
90.0
85.0
80.0
75.0
70.0
65.0
60.0
55.0
50.0
PB-3'-S
PB-3'-N
A46-3'-S
A46-3'-N
BF-3'-S
BF-3'-N
CR-3'-S
CR-3'-N
TR-3'-S
TR-3'-N
A65-3'-S
A65-3'-N
A68-3'-S
A68-3'-N
A70-3'-S
A70-3'-N
Average for existing linkers
Average for novel linkers

Fig. 7−1

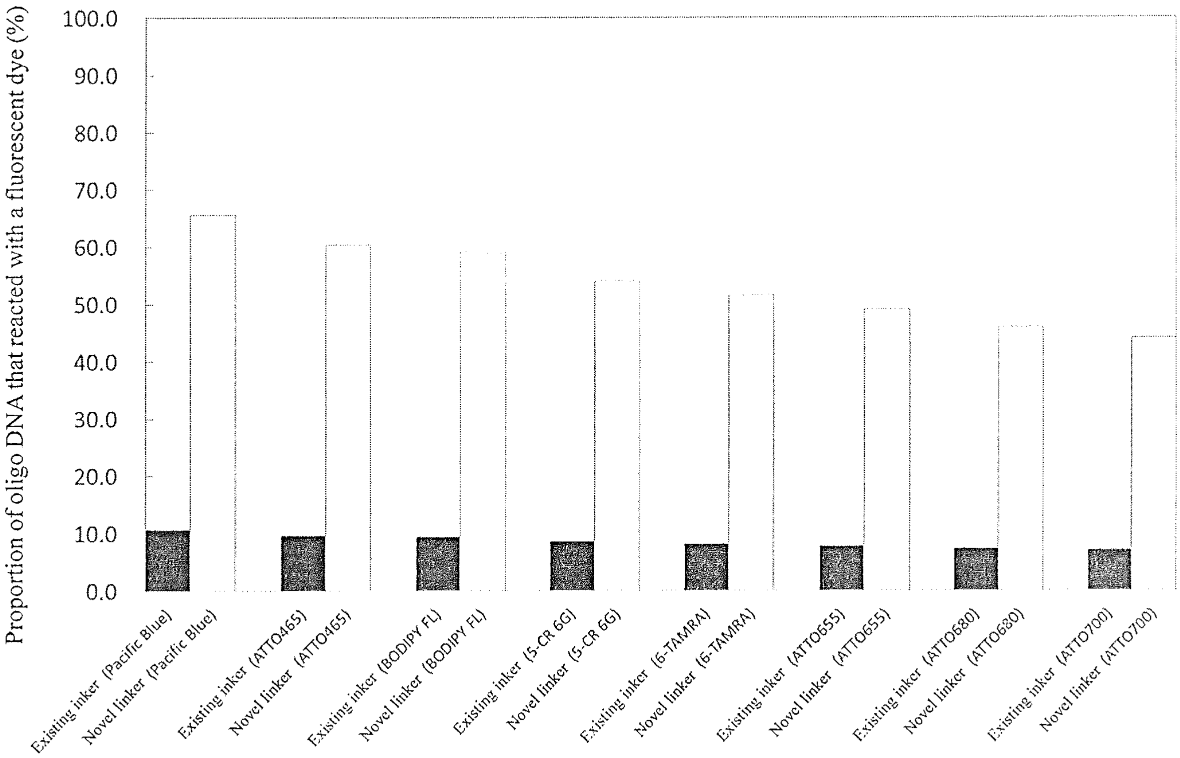
Proportion of oligo DNA that reacted with a fluorescent dye (%)
100.0
90.0
80.0
70.0
60.0
50.0
40.0
30.0
20.0
10.0
0.0
Existing inker (Pacific Blue)
Novel linker (Pacific Blue)
Existing inker (ATTO465)
Novel linker (ATTO465)
Existing inker (BODIPY FL)
Novel linker (BODIPY FL)
Existing inker (5-CR 6G)
Novel linker (5-CR 6G)
Existing inker (6-TAMRA)
Novel linker (6-TAMRA)
Existing inker (ATTO655)
Novel linker (ATTO655)
Existing inker (ATTO680)
Novel linker (ATTO680)
Existing inker (ATTO700)
Novel linker (ATTO700)

Fig. 7−2

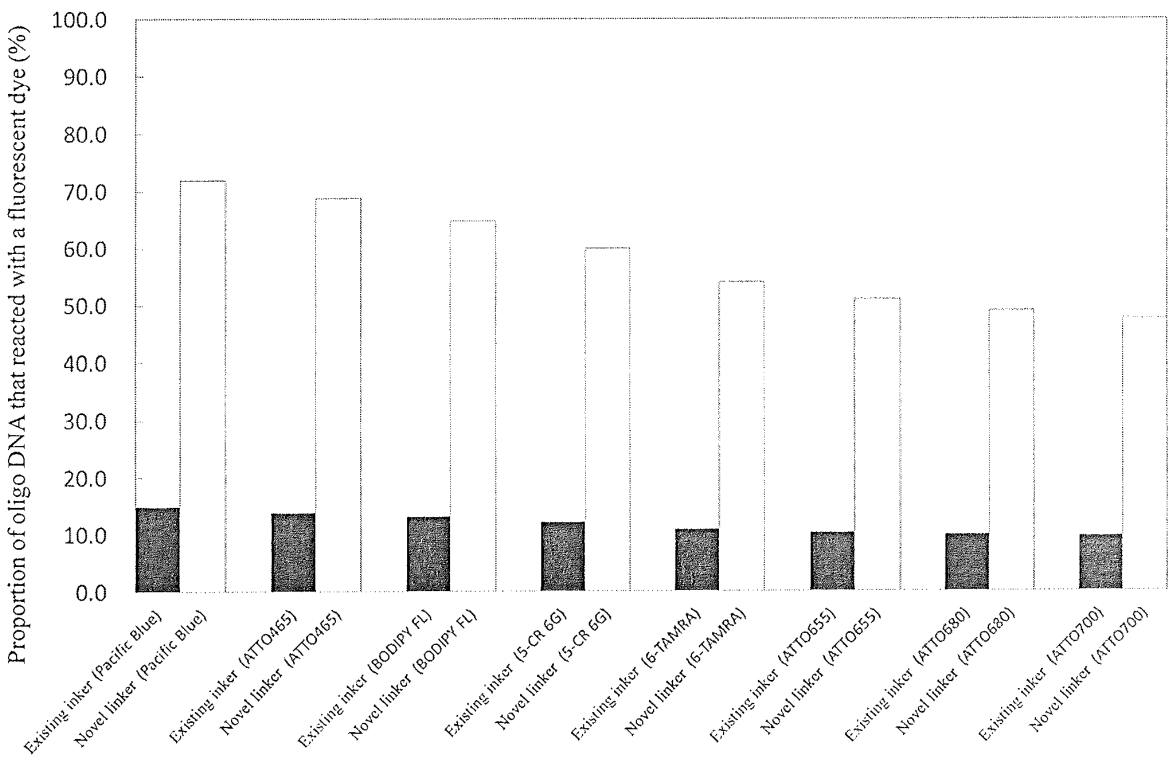
Proportion of oligo DNA that reacted with a fluorescent dye (%)
100.0
90.0
80.0
70.0
60.0
50.0
40.0
30.0
20.0
10.0
0.0
Existing inker (Pacific Blue)
Novel linker (Pacific Blue)
Existing inker (ATTO465)
Novel linker (ATTO465)
Existing inker (BODIPY FL)
Novel linker (BODIPY FL)
Existing inker (5-CR 6G)
Novel linker (5-CR 6G)
Existing inker (6-TAMRA)
Novel linker (6-TAMRA)
Existing inker (ATTO655)
Novel linker (ATTO655)
Existing inker (ATTO680)
Novel linker (ATTO680)
Existing inker (ATTO700)
Novel linker (ATTO700)

**INTERNATIONAL SEARCH REPORT**

International application No.
PCT/JP2018/019095

A. CLASSIFICATION OF SUBJECT MATTER
Int. Cl. C12Q1/6876(2018.01)i, C12N15/09(2006.01)i

According to International Patent Classification (IPC) or to both national classification and IPC

B. FIELDS SEARCHED
Minimum documentation searched (classification system followed by classification symbols)
Int. Cl. C12Q1/68-1/70, C12N15/00-15/90

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/MEDLINE/EMBASE/BIOSIS/WPIDS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y | JP 2001-286300 A (JAPAN BIOINDUSTRY ASSOCIATION) 16 October 2001, examples 1, 3 & US 2001/0000148 A1 (example 1, 3) & EP 1046717 A2 | 1-7 |
| Y<br>A | JP 2007-28993 A (NATIONAL INSTITUTE OF ADVANCED INDUSTRIAL SCIENCE AND TECHNOLOGY) 08 February 2007, claims, examples, all drawings, paragraph [0070] & US 2008/0227968 A1 (claims, examples, fig., paragraph [0071]) & WO 2007/013190 A1 & EP 1908829 A1 | 1, 3, 5-7<br>2, 4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19.07.2018 | 31.07.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/019095

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | WO 2013/024694 A1 (NATIONAL INSTITUTE OF ADVANCED INDUSTRIAL SCIENCE AND TECHNOLOGY) 21 February 2013, claims, examples, all drawings, paragraph [0048] & US 2014/0187724 A1 (claims, examples, fig., paragraph [0059]) & EP 2749566 A1 | 2, 4-7<br>1, 3 |
| A | WO 2012/173274 A1 (NIPPON STEEL & SUMIKIN ECO TECH CORP.) 20 December 2012, examples, all drawings & US 2014/0248611 A1 (examples, fig.) & EP 2722388 A1 | 1-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4336820 B
- JP 2017105202 A


### Non-patent literature cited in the description


- **HOLLAND PM ; ABRAMSON RD ; WATSON R ; GELFAND DH.** Detection of specific polymerase chain reaction product by utilizing the 5'----3' exonuclease activity of Thermus aquaticus DNA polymerase. *Proc Natl Acad Sci USA.,* 15 August 1991, vol. 88 (16), 7276-80
- **TYAGI S ; KRAMER FR.** Molecular beacons: probes that fluoresce upon hybridization. *Nat Biotechnol.,* March 1996, vol. 14 (3), 303-8
- **KURATA S ; KANAGAWA T ; YAMADA K ; TORIMURA M ; YOKOMAKU T ; KAMAGATA Y ; KURANE R.** Fluorescent quenching-based quantitative detection of specific DNA/RNA using a BODIPY((R)) FL-labeled probe or primer. *Nucleic Acids Res.,* 15 March 2001, vol. 29 (6), E34
- **TORIMURA M ; KURATA S ; YAMADA K ; YOKOMAKU T ; KAMAGATA Y ; KANAGAWA T ; KURANE R.** Fluorescence-quenching phenomenon by photoinduced electron transfer between a fluorescent dye and a nucleotide base. *Anal Sci.,* January 2001, vol. 17 (1), 155-60
- *Tetrahedron Letters,* 1981, vol. 22, 1859-1862
- *Nucleic Acids Research,* 1986, vol. 14, 6227-6245
- *Nature Biotechnology,* 1996, vol. 14, 303-308
- *Applied and Environmental Microbiology,* 1997, vol. 63, 1143-114
- *Nucleic Acids Research,* 1996, vol. 24, 4532-4535
- *Analytical Biochemistry,* 1998, vol. 225, 32-38
- *Analytical Biochemistry,* 1989, vol. 183, 231-244
- **YODOSHA.** *Jikken Igaku (Laboratory Medicine),* 1997, vol. 15 (7), 46-51

SEQUENCE LISTING

<110> Nippon Steel & Sumikin Eco-Tech Corporation

<120> Novel fluorescent quenching probes for measuring nucleic acids

<130> FP-236PCT

<150> JP 2017-105202
<151> 2017-05-29

<160> 4

<170> PatentIn version 3.5

<210> 1
<211> 17
<212> DNA
<213> Artificial Sequence

<220>
<223> Probe

<400> 1
cctacgggag gcagcag 17

<210> 2
<211> 17
<212> DNA
<213> Artificial Sequence

<220>
<223> Probe

<400> 2
ctgctgcctc ccgtagg 17

<210> 3
<211> 17
<212> DNA
<213> Artificial Sequence

<220>
<223> Probe

<400> 3
aaggaggtga tccagcc 17

<210> 4
<211> 21
<212> DNA
<213> Artificial Sequence

<220>
<223> Probe

<400> 4
aggccggccc ttgactttcc t 21